# FASCICULE DE BREVET EUROPEEN

(11) **EP 1 991 140 B1**
(45) Date de publication et mention de la délivrance du brevet: **07.04.2010**
(21) Numéro de dépôt: 07731056.3
(22) Date de dépôt: 27.02.2007
(51) Int. Cl.: A61B 17/30, B25B 9/02, A45D 26/00

(54) **PROCEDE DE FABRICATION DE POINTES DE PINCEMENT DE PINCES DE MICROCHIRURGIE A USAGE UNIQUE ET PINCES DE MICROCHIRURGIE MUNIES DE TELLES POINTES DE PINCEMENT**
VERFAHREN ZUR HERSTELLUNG VON EINWEG-PINZETTENSPITZEN FÜR MIKROCHIRURGISCHE PINZETTEN UND MIKROCHIRURGISCHE PINZETTEN MIT SOLCHEN SPITZEN
METHOD FOR PRODUCING DISPOSABLE TWEEZER TIPS FOR MICROSURGERY TWEEZERS AND MICROSURGERY TWEEZERS PROVIDED WITH SUCH TIPS

(30) Priorité: 28.02.2006 FR 0601766
(43) Date de publication de la demande: 19.11.2008
(73) Titulaire: Becton, Dickinson and Company, Inc., Franklin Lakes, NJ 07417-1880 (US)
(72) Inventeur: Andre, Jean-Marie, 13001 Marseille (FR)
(74) Mandataire: Brédeville, Odile Marie
(86) Numéro de dépôt international: PCT/FR2007/000354
(87) Numéro de publication internationale: WO 2007/099225

(56) Documents cités:
- FR-A- 2 863 872
- GB-A- 2 239 417
- US-A- 4 452 106
- US-A- 5 921 990

## Description

La présente invention concerne un procédé de fabrication de pointes de pincement de microchirurgie ophtalmologique à usage unique, dédiées à la chirurgie de la cataracte. Elle vise également les pointes ou dents de pincement résultant de la mise en oeuvre de ce procédé, et les pinces de microchirurgie munies de telles pointes de pincement.

D'une manière générale, l'invention se rapporte à un procédé de fabrication de pointes de pincement de pinces de microchirurgie à usage unique du genre comportant deux branches reliées l'une à l'autre par l'une de leurs extrémités pour former la partie proximale de préhension de la pince et dont les extrémités opposées sont constituées par des pointes de pincement susceptibles d'être rapprochées élastiquement l'une de l'autre et formant la partie active de ladite pince.

Les opérations de microchirurgie ophtalmologique, et notamment les opérations de la cataracte, exécutées sous contrôle de microscopes, nécessitent l'utilisation de pinces de grande précision, voire d'ultra précision, pourvues de pointes de pincement extrêmement fines. Par exemple, de telles pointes de pincement peuvent présenter, dans leur partie distale, une section très réduite de 0,25 x 0,25 mm et des micro-dents de préhension extrêmement fines d'une longueur de l'ordre de 0,1 mm.

II s'agit d'une catégorie de pinces de grande précision très spécifique pour laquelle l'homme du métier ayant une grande expérience de ce type d'instrumentation de microchirurgie est différent de l'homme du métier spécialisé dans l'utilisation ou la fabrication d'autres genres de pinces de chirurgie.

Jusqu'à présent, l'homme de l'art (chirurgien ophtalmologique) était convaincu que de tels instruments de grande précision devaient être fabriqués dans un matériau d'une grande dureté, ( voir normes Afnor NF S94-090 Avril 2001 : Matériel pour instruments de chirurgie : Aciers inoxydables martensitiques à durcissement par précipitation, austenitique et austéno-ferritiques ) de sorte à éviter toute déformation de leurs pointes de pincement et l'épointement des micro-dents de celles-ci.

Dans ces conditions, actuellement, ces instruments de grande précision sont le plus souvent fabriqués en un métal dur et inoxydable, par des procédés artisanaux et manuels, ce qui les rend très coûteux. Ces instruments généralement exécutés en aciers martensitiques ,en aciers austénitiques ou encore en aciers austéno-ferritiques, (notamment en inox 302) dénommés "aciers chirurgicaux" dans les milieux professionnels, en raison de leurs bonnes caractéristiques de dureté et de leur biocompatibilité. Leur coût de fabrication élevé est un obstacle majeur s'opposant à ce que ces instruments soient proposés comme instruments "jetables", alors que leur non-réutilisation serait sanitairement et économiquement souhaitable. En effet, la stérilisation et/ou la décontamination obligatoire des instruments chirurgicaux réutilisables et leur conservation à l'état aseptisé, entre deux utilisations successives, imposent d'importants investissements en personnel qualifié, en matériel et en temps de travail. D'autre part, ces instruments deviennent inutilisables dès que leurs micro-dents sont quelque peu déformées ou émoussées et ne présentent plus la précision de fonctionnement indispensable.

Pour abaisser le prix de revient de ces instruments de sorte à en faire des articles à usage unique, il faut parvenir à en mécaniser la production.

Pour répondre à cet objectif, on a déjà proposé une pince chirurgicale jetable entièrement réalisée par moulage sous pression d'une matière thermoplastique et se présentant sous forme d'une seule pièce monobloc.

L'idée de réaliser les pinces de microchirurgie oculaire en matière plastique thermoformée peut paraître d'emblée très séduisante, en raison du caractère économique de ce mode de production. Cependant, les praticiens ont pu constater que si ce matériau convient pour la fabrication des manches ou parties proximales des branches des instruments, il ne présente pas les caractéristiques physiques indispensables de finesse et de dureté propres au métal, dans la partie distale active (pointes de pincement des branches) desdits instruments, pour une utilisation en microchirurgie.

Pour remédier aux inconvénients susmentionnés des instruments chirurgicaux entièrement réalisés en métal ou des instruments chirurgicaux complètement exécutés en matière plastique, on a déjà proposé de réaliser certains de ces instruments avec une partie active métallique surmoulée par une matière plastique injectée sous pression constituant le manche ou partie proximale de préhension desdits instruments.

Le document FR-2863872 décrit, par exemple, une pince chirurgicale, en particulier une pince de microchirurgie ophtalmologique comprenant, d'une part, une partie de préhension moulée d'une seule pièce en matière plastique et formée de deux bras reliés l'un à l'autre par l'une de leurs extrémités pour former la partie proximale de la pince et, d'autre part, une partie de pincement constituée par des pointes ou dents métalliques insérées dans les extrémités distales des bras, lors du moulage de ladite partie de préhension.

Grâce à un tel mode de fabrication, il est possible de réaliser des pinces chirurgicales de façon plus économique permettant d'envisager de les réserver à un usage unique.

Toutefois, le coût de telles pinces chirurgicales composites reste relativement élevé car les pointes sont réalisées en aciers inoxydables martensitiques, en aciers austénitiques ou encore en aciers austéno-ferritiques, (voir normes Afnor NF S94-090 Avril 2001) dont les caractéristiques mécaniques, en particulier la dureté, ne sont compatibles avec aucun procédé mécanisé permettant d'obtenir la précision requise ; ces pointes ultra précises sont donc obligatoirement façonnées une à une, à la main, artisanalement, ce qui, bien sûr, en augmente le coût.

Un but de la présente invention est donc la conception d'un procédé autorisant pour la première fois une mécanisation complète de la fabrication des pointes ou dents de pincement des pinces de chirurgie les plus précises actuellement existantes, c'est-à-dire des pinces de microchirurgie oculaire, ceci permettant donc pour la première fois une production de masse de tels instruments grâce à laquelle le prix de revient de ces derniers peut être très sensiblement réduit.

Plus précisément, l'invention vise à obtenir des pinces de microchirurgie à usage unique, obligatoirement jetables et non réutilisables.

Pour cela, l'invention a conduit à rechercher un matériau autre que les aciers de grande dureté habituels et présentant à la fois :
- des caractéristiques de résistance à la corrosion compatibles avec leur utilisation en tant qu'instrument de chirurgie à usage unique ,
- des caractéristiques de bio-compatibilité grâce auxquelles les pointes de pincement sont utilisables lors d'interventions de microchirurgie ;
- des caractéristiques de tendreté permettant le découpage de pièces extrêmement fines et précises, sur une presse à découper ;
- des caractéristiques de rigidité ou de dureté grâce auxquelles les pointes de pincement soient aptes à remplir de manière très fiable leurs fonctions lors de la première opération de microchirurgie ;
- des caractéristiques de tendreté qui, du fait de la finesse importante des pointes de pincement munies de dents de préhension de 0,1 mm de longueur, font que la durée de vie de ces mêmes dents est limitée, et que, bien que totalement fonctionnelles et présentes au cours de la première intervention de microchirurgie, ces micro-dents de préhension s'émoussent ensuite progressivement, sans perte de matière, jusqu'à devenir inexistantes et donc non fonctionnelles à la fin de l'intervention de microchirurgie. Cette caractéristique interdit une réutilisation répétée, non voulue par le fabricant et le praticien, de ces pinces destinées à l'usage unique.
- un prix de revient réduit autorisant la fabrication de pinces de microchirurgie à usage unique.

Cette recherche a conduit à une solution qui va à l'encontre de l'opinion prévalant parmi les spécialistes et suivant laquelle les pointes de pincement des pinces de microchirurgie doivent obligatoirement être exécutées en acier inoxydable dit « chirurgical » qui est un acier inoxydable spécifique et d'une grande dureté, ( voir normes Afnor NF S94-090 Avril 2001 : Matériel pour instruments de chirurgie : Aciers inoxydables martensitiques à durcissement par précipitation, austénitiques et austéno-ferritiques)

Selon l'invention, les objectifs susmentionnés sont atteints grâce à un procédé suivant lequel les pointes de pincement des pinces de microchirurgie sont réalisées par découpage métallique de haute précision dans un outil à suivre de découpage de haute précision actionné par presse. Le métal découpé doit posséder un coefficient de résistance à la traction compris entre 350 et 550 Rm N / mm², il s'agit, de manière avantageuse, selon l'invention, d'une bande ou plaque d'acier inoxydable ferritique 430 encore appelé inox 430.

Ce procédé permet de réaliser des pointes de pincement de pinces de microchirurgie ophtalmologique pour la chirurgie de la cataracte, de manière entièrement automatique, ces dernières présentant la finesse souhaitable (par exemple une largeur et une épaisseur de l'ordre de 0,25 mm dans leur portion distale active, et une dent de préhension d'une longueur de 0,1 mm), une rigidité suffisante pour remplir leur fonction avec l'efficacité et la sécurité indispensables et une tendreté entraînant l'épointement de leurs dents de préhension au terme de la durée d'une intervention de microchirurgie oculaire telle qu'une opération de la cataracte. En outre, ces dents de pincement sont exécutées dans un métal inoxydable bio-compatible.

Les buts, caractéristiques et avantages ci-dessus, et d'autres encore, ressortiront mieux de la description qui suit et des dessins annexés dans lesquels :
La figure 1 est une vue en perspective d'un exemple de réalisation d'une pince de microchirurgie oculaire munie de pointes ou dents de pincement à la fabrication desquelles peut être appliqué le procédé de l'invention.

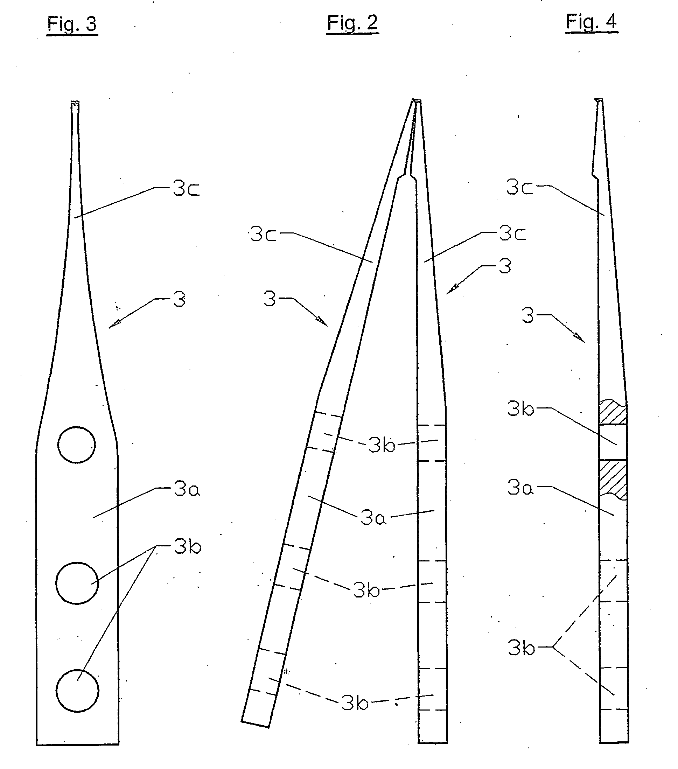

La figure 2 est une vue de face et à plus grande échelle, d'un couple de pointes de pincement représentées en position rapprochée..
La figure 3 est une vue de côté (face interne) de l'une de ces pointes de pincement.
La figure 4 est une vue de face, avec arrachement partiel, d'une pointe de pincement.
La figure 5 est une vue en perspective, à échelle très agrandie, des micro-dents de préhension dont sont pourvues les extrémités des pointes de pincement.

On se reporte auxdits dessins pour décrire un exemple intéressant bien que nullement limitatif, de mise en oeuvre du procédé et de réalisation des dents de pincement selon l'invention.

On décrit ci-après une application particulièrement avantageuse de l'invention à la réalisation de pinces chirurgicales et, plus précisément, de pince de microchirurgie oculaire.

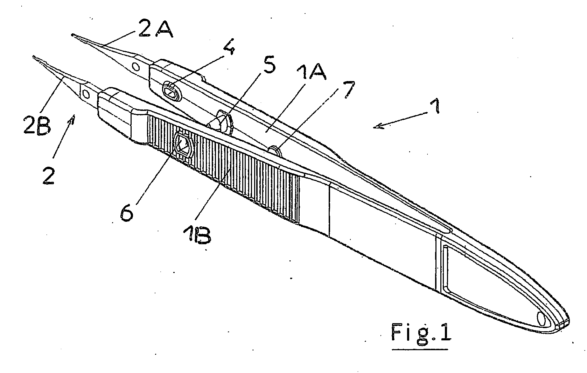

La figure 1 représente un exemple de réalisation de pince de microchirurgie oculaire munie de pointes de pincement à la fabrication desquelles est applicable le procédé de l'invention.

De telles pinces composites décrites dans le document FR-2863872, comprennent : d'une part, une partie de préhension 1 exécutée en matière plastique et formée de deux branches ou bras 1A, 1 B reliés l'un à l'autre par l'une de leurs extrémités pour constituer le manche ou partie proximale de la pince, et, d'autre part, une partie de pinçage 2 constituée par des pointes métalliques de pincement 3 implantées dans les extrémités distales desdits bras.

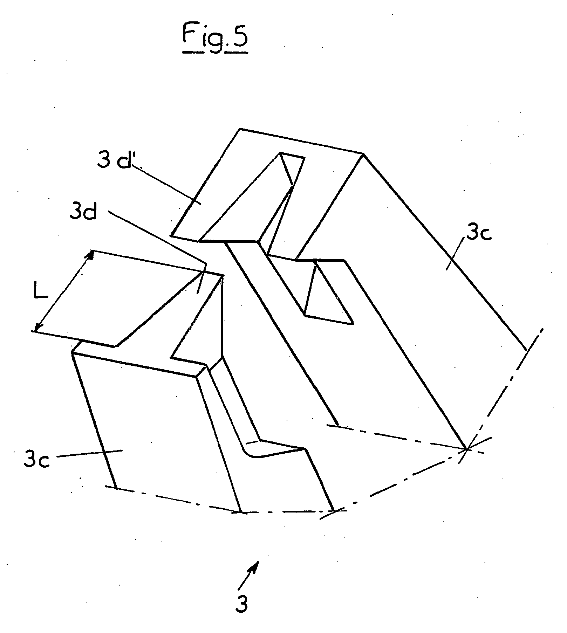

Les pointes de pincement 3 réalisées comportent, de façon connue en soi : d'une part, une partie proximale 3a appelée à être ancrée dans l'extrémité distale des bras d'une pince, cette partie proximale 3a étant munie de trous 3b la traversant dans son épaisseur pour coopérer à la stabilité de cet ancrage, et, d'autre part, une partie distale de pincement 3c munie d'une dent de préhension 3d ou 3d' (figure 5).

Selon le procédé de l'invention, les pointes de pincement 3 munies de leur dent de préhension 3d ou 3d' sont découpées et façonnées sous presse à découper munie d'outils de découpe à suivre appropriés dans une plaque ou bande de métal inoxydable et bio-compatible. Le métal découpé doit posséder un coefficient de résistance à la traction compris entre 350 et 550 Rm N / mm². II s'agit, de manière avantageuse, d'une bande ou plaque d'acier inoxydable ferritique 430 encore appelé inox 430.

De façon intéressante, on utilise une bande ou plaque d'acier inoxydable ferritique 430 présentant une épaisseur comprise entre 0.8 mm et 1 mm (0,8 mm et 1 mm inclus), pour le découpage et le façonnage sur presse des pointes de pincement 3.

La partie proximale 3a des pointes de préhension 3 présente une épaisseur et une largeur constantes sur toute sa longueur, par exemple une épaisseur de 1 mm et une largeur de 3 mm, tandis que la partie distale présente une section s'amincissant en direction de son extrémité, de sorte à présenter, dans sa partie terminale, une section très réduite, par exemple de l'ordre de 0,25 x 0,25 mm. D'autre part, la partie distale 3c de chaque pointe de pincement 3 est munie d'une dent de préhension 3d ou 3d' d'une longueur L de l'ordre de 0,1 mm et orientée en direction de la dent de préhension 3d' ou 3d dont est munie la partie distale 3c de l'autre pointe de pincement 3.

Les presses à découper et les outils de découpe à suivre utilisables pour la mise en oeuvre du procédé de l'invention sont connus en soi.

L'utilisation conjuguée de l'inox ferritique 430 et de la presse permet un découpage et un façonnage de haute précision des dents ou pointes de pincement.

On souligne ici que l'acier inoxydable ferritique 430 n'a pas été utilisé jusqu'à présent pour la réalisation d'instruments de chirurgie, en raison de son degré de malléabilité trop élevé considéré comme un défaut l'excluant de l'éventail des matériaux utilisables pour la fabrication de tels instruments ( voir normes Afnor NF S94-090 Avril 2001 :
Matériel pour instruments de chirurgie: Aciers inoxydables martensitiques à durcissement par précipitation, austénitiques et austéno-ferritiques ).

Selon la présente invention, ce préjugé a été écarté et il a été considéré que la relative malléabilité de l'inox ferritique 430, n'était pas rédhibitoire pour son usage dans la fabrication de pointes de pincement de pinces de microchirurgie et notamment de pinces de microchirurgie ophtalmologique destinées à la manipulation de tissus ou implants mous de très petites dimensions (par exemple manipulation du cristallin, mise en place d'implants cristalliniens, ...). Dans de telles applications, les pointes de pincement des pinces, bien que présentant un degré de malléabilité relativement élevé, possèdent aussi un coefficient de rigidité ou dureté suffisant pour permettre la manipulation de tissus mous avec l'efficacité et la sécurité indispensables.

Ce procédé permet une fabrication industrielle des pointes ou dents de pincement des pinces de précision composites, notamment des pinces chirurgicales composites et, par conséquent, une production de masse de tels instruments, en particulier des pinces de microchirurgie ophtalmologique à usage unique.

L'invention concerne également les pinces de précision composites et notamment les pinces chirurgicales composites, et plus particulièrement les pinces, de microchirurgie oculaire ou autre, munies de dents ou pointes de pincement métalliques du genre précédemment décrit et résultant de la mise en oeuvre du procédé exposé ci-dessus.

## Revendications

1. Procédé de fabrication de pointes de pincement des pinces de microchirurgie ophtalmologique pour la chirurgie de la cataracte, à usage unique, en particulier du genre comprenant, d'une part, une partie de préhension moulée d'une seule pièce en matière plastique et formée de deux bras reliés l'un à l'autre par l'une de leurs extrémités pour former la partie proximale de la pince et, d'autre part, une partie de pincement constituée par des pointes métalliques insérées dans les extrémités distale desdits bras, lors du moulage de la partie de préhension, **caractérisé en ce que** lesdites pointes de pincement (3) munies de leurs dents de préhension (3d, 3d') sont découpées et façonnées sur presse à découper munie d'outils de découpe à suivre appropriés connus en soi, à partir d'une bande ou plaque d'acier ferritique inoxydable 430 possédant un coefficient de résistance à la traction compris entre 350 et 550 Rm N / mm².

2. Procédé de fabrication suivant la revendication 1, **caractérisé en ce que** les pointes de pincement (3) munies de leur dent de préhension (3d, 3d') sont découpées et façonnées sur presse à découper, à partir d'une bande ou plaque d'acier ferritique inoxydable 430 présentant une épaisseur comprise entre 0.8 mm et 1 mm, ( 0 .8 mm et 1 mm inclus).

3. Pointes de pincement pour pinces de microchirurgie ophtalmologique pour la chirurgie de la cataracte, à usage unique, du genre comportant, d'une part, une partie proximale (3a) appelée à être ancrée dans l'extrémité distale de l'un des bras d'une pince de microchirurgie et présentant, par exemple, une épaisseur de l'ordre de 1 mm et une largeur dé l'ordre de 3 mm, et, d'autre part, une partie distale de pincement (3c) présentant une section s'amincissant en direction de son extrémité, de sorte à présenter, dans sa partie terminale, une section très réduite, par exemple de l'ordre de 0,25 x 0,25 mm, ladite partie terminale étant munie d'une dent de préhension (3d ou 3d') d'une longueur de l'ordre de 0,1 mm, **caractérisées en ce qu'**elles sont obtenues par la mise en oeuvre du procédé 'selon l'une quelconque des revendications 1 ou 2.

4. Pinces de microchirurgie ophtalmologique à usage unique du genre comportant, d'une part, une partie de préhension (1) exécutée en matière plastique et formée de deux bras (1A, 1B) reliés l'un à l'autre par l'une de leurs extrémités pour constituer la partie proximale de la pince et, d'autre part, une partie de pinçage (2) constituée par des pointes métalliques de pincement (3) implantées dans les extrémités distales desdits bras (1A, 1B), **caractérisées en ce que** les dites pinces sont munies de pointes de pincement (3) selon la revendication 3, ou exécutées selon le procédé de l'une des revendications 1 ou 2.

## Claims

1. Method for producing tweezer tips of disposable ophthalmological microsurgery tweezers for cataract surgery, in particular of the type comprising, on the one hand, a grip part moulded from a single piece of plastic and formed by two arms that are connected to each other via one of their ends in order to form the proximal part of the tweezers, and, on the other hand, a tweezer part composed of metal tips that are inserted into the distal ends of said arms during the moulding of the grip part, **characterized in that** said tweezer tips (3), provided with their gripping teeth (3d, 3d'), are cut and shaped, on a cutting press provided with appropriate cutting tools known per se, from a band or plate of ferritic stainless steel 430 having a tensile strength coefficient of between 350 and 550 Rm N/mm².

2. Method of production according to Claim 1, **characterized in that** the tweezer tips (3) provided with their gripping teeth (3d, 3d') are cut and shaped, on a cutting press, from a band or plate of ferritic stainless steel 430 having a thickness of between 0.8 mm and 1 mm (including 0.8 mm and 1 mm).

3. Tweezer tips for disposable ophthalmological microsurgery tweezers for cataract surgery, of the type comprising, on the one hand, a proximal part (3a) designed to be anchored in the distal end of one of the arms of microsurgery tweezers and having, for example, a thickness of the order of 1 mm and a width of the order of 3 mm, and, on the other hand, a distal tweezer part (3c) having a cross section that narrows in the direction of the end thereof, so as to have a greatly reduced cross section at its end part, for example of the order of 0.25 x 0.25 mm, said end part being provided with a gripping tooth (3d or 3d') with a length of the order of 0.1 mm, **characterized in that** they are obtained by implementing the method according to either of Claims 1 and 2.

4. Disposable ophthalmological microsurgery tweezers, of the type comprising, on the one hand, a grip part (1) made of plastic and formed by two arms (1A, 1B) that are connected to each other via one of their ends in order to form the proximal part of the tweezers, and, on the other hand, a tweezer part (2) composed of metal tweezer tips (3) that are implanted in the distal ends of said arms (1A, 1B), **characterized in that** said tweezers are provided with tweezer tips (3) according to Claim 3 and/or are produced according to the method of one of Claims 1 and 2.

## Patentansprüche

1. Verfahren zur Herstellung von Einweg-Greifspitzen der ophthalmologischen mikrochirurgischen Pinzetten für die Katarakt-Chirurgie, insbesondere von der Art, die einerseits einen Griffteil, der aus einem Stück aus Kunststoff geformt ist und von zwei zur Bildung des proximalen Teils der Pinzette miteinander über ihre Enden verbundenen Armen gebildet wird, und andererseits einen Greifteil enthält, der aus metallischen Spitzen besteht, die beim Formen des Griffteils in die distalen Enden der Arme eingefügt werden, **dadurch gekennzeichnet, dass** die mit ihren Greifzähnen (3d, 3d') versehenen Greifspitzen (3) ausgehend von einem Band oder einer Platte aus rostfreiem Ferritstahl 430 mit einem Zugwiderstandskoeffizient zwischen 350 und 550 Rm N/mm² auf einer mit an sich bekannten, geeigneten Folgeschneidwerkzeugen versehenen Schneidpresse ausgeschnitten und bearbeitet werden.

2. Herstellungsverfahren nach Anspruch 1, **dadurch gekennzeichnet, dass** die mit ihren Greifzähnen (3d, 3d') versehenen Greifspitzen (3) ausgehend von einem Band oder einer Platte aus rostfreiem Ferritstahl 430 mit einer Dicke zwischen 0,8 mm und 1 mm, (einschließlich 0,8 mm und 1 mm) auf einer Schneidpresse ausgeschnitten und bearbeiten werden.

3. Einweg-Greifspitzen für ophthalmologische mikrochirurgische Pinzetten für die Katarakt-Chirurgie, von der Art, die einerseits einen proximalen Teil (3a), der dazu bestimmt ist, im distalen Ende eines der Arme einer mikrochirurgischen Pinzette verankert zu werden und zum Beispiel eine Dicke in der Größenordnung von 1 mm und eine Breite in der Größenordnung von 3 mm hat, und andererseits einen distalen Greifteil (3c) enthält, der einen sich in Richtung seines Endes verjüngenden Querschnitt hat, um in seinem Endbereich einen stark reduzierten Querschnitt zu haben, zum Beispiel in der Größenordnung von 0,25 x 0,25 mm, wobei der Endbereich mit einem Greifzahn (3d oder 3d') einer Länge in der Größenordnung von 0,1 mm versehen ist, **dadurch gekennzeichnet, dass** sie durch die Anwendung des Verfahrens nach einem der Ansprüche 1 oder 2 erhalten werden.

4. Ophthalmologische mikrochirurgische Einweg-Pinzetten von der Art, die einerseits einen Griffteil (1), der aus Kunststoff hergestellt ist und von zwei Armen (1A, 1B) gebildet wird, die an einem ihrer Enden miteinander verbunden sind, um den proximalen Teil der Pinzette zu bilden, und andererseits einen Greifteil (2) aufweisen, der aus metallischen Greifspitzen (3) besteht, die in die distalen Enden der Arme (1A, 1B) eingesetzt sind, **dadurch gekennzeichnet, dass** die Pinzetten mit Greifspitzen (3) nach Anspruch 3 versehen oder nach dem Verfahren eines der Ansprüche 1 oder 2 hergestellt sind.
